(19)

Europäisches Patentamt
European Patent Office
Office européen des brevets

(11) **EP 4 578 444 A1**

(12) **EUROPEAN PATENT APPLICATION**

(43) Date of publication:
02.07.2025 Bulletin 2025/27

(21) Application number: 23220681.3

(22) Date of filing: 29.12.2023

(51) International Patent Classification (IPC):
*A61K 31/015* (2006.01)    *A61K 8/31* (2006.01)
*A61P 17/00* (2006.01)    *A61P 17/04* (2006.01)
*A61Q 19/00* (2006.01)    *A61Q 19/10* (2006.01)

(52) Cooperative Patent Classification (CPC):
A61K 31/015; A61K 8/31; A61P 17/00;
A61P 17/04; A61Q 19/00; A61Q 19/007;
A61Q 19/10

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: SkyLab AG
1066 Epalinges (CH)

(72) Inventors:
• Belous, Elena Yur'evna
121309 Moscow (RU)
• Yarovaya, Alina Olegovna
109518 Moscow (RU)
• Filatov, Viktor Andreevich
119415 Moscow (RU)

(74) Representative: Glawe, Delfs, Moll
Partnerschaft mbB
Hopfenmarkt 33
20457 Hamburg (DE)

(54) **APPLICATION OF BETA-CARYOPHYLLENE FOR TARGETED EPIGENETIC REGULATION AND POTENTIAL PREVENTION OF WATER-OSMOTIC CONDITIONS OF THE MUCOUS MEMBRANES AND EPITHELIA**

(57)    The invention relates to a composition comprising 0.01-0.3 wt.% $\beta$-caryophyllene and the use of said composition as a medicament, including for use in the treatment of a disease associated with a deficiency of aquaporins type 3 and/or aquaporins type 5.

Evaluation of beta-caryophyllene cytotoxicity on keratinocytes

**Fig. 1**

EP 4 578 444 A1

**Description**

PERTINENT ART

The invention relates to pharmaceuticals, nutraceuticals and reverse cosmeceuticals and represents beta-caryophyllene, pharmaceutical and cosmeceutical products containing beta-caryophyllene and their use for body mucous membrane and epithelial cell care for the purpose of prevention and epigenetic regulation of diseases associated with a lack of aquaporins types 3 and 5. The invention relates to beta-caryophyllene of plant origin from essential oils, fruits, seeds, leaves, grass and/or flowers, where the mass content of the component equivalent to the active substance is 0.01-0.3 wt.%.

Beta-caryophyllene is intended for inclusion in pharmaceutical products and perfumes and cosmetics for mucous membrane and epidermal cell care, the use of which makes it possible to epigenetically increase the quantity of type 3 (AQP 3) and type 5 (AQP5) aquaporins in young keratinocytes of the skin and scalp epidermis, mucous epithelium, secretory epithelium, nasal mucosa and respiratory tract epithelium, regulate water-osmotic balance and correct disease-associated conditions. The component is of natural origin, biodegradable, has a safe effect on sensitive skin and can be used for the manufacture of various products for epidermis and mucous membrane care, such as body lotions, face cleanser, face, body and hand skin care creams, face skin care serums, toothpastes, foam rinses, tooth cleaning foams, nasal mucosa ointments and creams, inhalations, liquid shower gels, scalp care shampoos, dandruff seborrheic dermatitis and xerosis shampoos. The use of beta-caryophyllene promotes epigenetic regulation of water-osmotic balance, prevention of mucous membrane and epidermis diseases and alleviation of disease-associated conditions by increasing the quantity of type 3 (AQP3) and type 5 (AQP5) aquaporins and creating isosmotic conditions.

BACKGROUND

Aquaporins are a group of proteins that play a key role in regulation of water transport across cell membranes. These proteins have the ability to control the penetration of water through cell walls, which is of great importance for the maintenance of body water balance. Recent studies have shown that aquaporins represent a new and understudied target for the prevention and treatment of various diseases. Moreover, aquaporins may contribute to the regulation of inflammation and wound healing, which make them potentially important for skin care and the treatment of human mucosal diseases [1,2].

Aquaporin-3 (AQP3) plays an important role in the maintenance of skin, nose mucous membrane and eye hydration. It participates in the transport of glycerol and water across cell membranes. AQP3 deficiency or dysfunction may be associated with various skin diseases, such as dry skin, atopic dermatitis, and psoriasis [3]. Aquaporin-5 (AQP5) is found in various tissues, including the skin and mucous membranes, where it plays an essential role in the regulation of hydration. AQP5 is involved in various diseases of the skin and mucous membranes, such as dry eye syndrome, xerostomia, as well as dermatitis, eczema, and psoriasis [4]. Research into these types of aquaporins may facilitate the development of new approaches to the treatment of various conditions associated with impaired hydration of the skin and mucous membranes.

In addition to the obvious need to prevent the loss of water from the epidermis and mucous membrane cells into the environment, these tissues themselves must maintain the correct water-osmotic balance in the viable cell layers. Aquaporins (AQPs) are a family of transmembrane channel proteins that are partly responsible for this function through transporting water and other fine solvents between and within cells [19]. In recent years, numerous members of the AQP protein family have been identified in various tissues of the body, and information about their properties and functions has been obtained. Although moisture balance is well known to be fundamental to skin health, the physiological mechanisms underlying the control of hydration, particularly in the epidermis, are not yet fully understood. Over the past 10 years, many studies have been carried out to understand the nature and regulation of the water gradient that exists in the epidermis layers. The membrane-associated pores called aquaporins are of paramount importance facilitating the passage of water and, in some cases, small molecules such as glycerol, urea and other low molecular weight molecules [20].

The spatial structure of aquaporin resembles a cylindrical channel through which water molecules move. Not only water, but ions and low molecular weight substances pass through it. The amino acids in the protein are arranged in such a way that the polarity of the electrostatic field they create is "switched" in the center of the molecule to the reverse one. Therefore, water molecules, having reached the middle of the channel, turn over so that their dipole moments in the upper and lower parts of the channel are directed in opposite sides. This reorientation prevents ions from leaking through the channel. Aquaporin holds even hydronium ions $H_3O^+$ (i.e. hydrated protons), the concentration of which determines the acidity of the medium.

The most abundant aquaporins in the skin and scalp epidermis are AQP3 and AQP5. These proteins, mostly expressed in all layers of epidermal keratinocytes [21], belong to the so-called group of aquaglyceroporins, which transport both water and moisturizing glycerol, and thus play an important role in the maintenance of hydration level in the mammalian skin epidermis [22]. AQP3 is involved in the regulation of keratinocyte differentiation [23,24] and many other processes related to skin hydration, barrier function, wound healing and cell migration [22,25-27]. Decreased AQP3 expression has also been demonstrated in skin diseases associated with increased transepidermal water loss and decreased stratum corneum hydration [25]. Moreover, recent reports show that AQP3 is decreased in several other skin diseases such as psoriasis [28] or diabetes-related diseases [29]. AQP3 content in human skin has also been reported to decrease with age and chronic sun exposure [30].

There are three types of aquaporins in the salivary glands: AQP1, AQP3 and AQP5. AQP3 is present on the basolateral membrane while AQP5 dwells on the apical membrane [31]. Aquaporin-5 (AQP5) is a water channel protein that is selectively expressed in respiratory tissues, secretory glands [32] and skin [33]. A study [34] showed that AQP5 plays a central role in salivary secretion, prevents excess water reabsorption and modulates cell shape and volume. A study [35] found that AQP5-deficient mice secrete a small volume of viscous hypertonic saliva after supramaximal stimulation with pilocarpine. They proposed that AQP5 contributes to the regulation of membrane water permeability and that it is also required to maintain proper osmolality of secreted saliva. The results of this study are consistent with at least two possible mechanisms for the occurrence of hyposalivation in mice lacking AQP5. AQP5 deficiency is associated with genetic diseases such as hereditary palmoplantar keratoderma [36], hidradenitis supurativa, psoriasis [37], and Sjögren's syndrome [38] (which has no clinically recommended treatment). Thus, AQP5 is required for transcellular water movement. Alternatively, targeted disruption of the AQP5 gene could alter the fluid and electrolyte balance of the entire animal resulting in a state of dehydration, which is known to suppress salivation.

The main cause of dryness is poor water-osmotic balance at the cellular level. Aquaporins improve this process by ensuring efficient transport of water across cell membranes. This deep action at the molecular level results in an increase in tissue moisture content, which in turn reduces the feeling of dryness. Application of knowledge about aquaporins in the development of new activity modulators can facilitate the development of new targeted therapy, effective pharmaceuticals and new cosmeceuticals, approaches to solving the problem of dryness, prevention and treatment of common diseases of the skin, mucous membranes of the body, nervous tissue and human secretory glands. Appropriate functional assays, including suitable cellular models and methods to accurately assess membrane permeability to water and solutes, are required to confirm aquaporin function and assess short-term regulation [39]. Currently, there is no targeted and epigenetic therapy for various human diseases mediated by a genetic or epigenetic decrease in the quantity of aquaporins 3 and 5 in body cells. Problems with the water-osmotic balance on the skin and mucous membranes can result in various diseases associated with tissue hydration.

Xerosis is a condition that reduces skin capacity due to decreased skin barrier function and increased transepidermal water loss (TEWL). The overall prevalence of xerosis in the world is estimated at 29-85%, i.e. it is observed in almost every second person [5]. The skin is a protective layer against physical and chemical damage and sources of infection. In general, young, well-hydrated skin is less susceptible to environmental stress, and itching and inflammation associated with dry skin are less likely to occur. Xerodermia also develops in specific genetic diseases, such as ichthyosis, Netherton's syndrome, atopic dermatitis, dandruff and seborrheic dermatitis, eczema, and psoriasis [6, 7-10]. Acquired dry skin can also be an accompanying symptom for a number of malignant diseases (gastrointestinal tract events, Hodgkin's disease, lymphoma), infectious pathology (HIV/AIDS, viral hepatitis), mental disorders (psychogenic anorexia), endocrine dysfunction (thyroiditis, diabetes mellitus), renal failure, and is observed in patients with various diets, hypovitaminosis, malabsorption syndrome, etc. [11]. This condition is influenced by aging mechanisms, environmental factors, a decrease in water content of the epidermis and mucous membrane stratum corneum, a decrease in the content of lipids and natural moisturizing factors in keratinocytes, as well as a decrease in the collagen content in the extracellular matrix [12]. In some dermatological diseases, desquamation and excessive TEWL are the main clinical manifestation [13]. A deeper understanding of the xerosis mechanism provides a fresh look at the principle of action of modern substances and opens up opportunities for the development of new treatment methods aimed at preventing and correcting the epigenetic mechanisms of xerosis and maintaining the water-osmotic state of mucosal and epidermal cells. Currently, there are no targeted or epigenetic therapies that eliminate these conditions.

Xerostomia is a condition that is characterized by dryness of the mucous membranes and secretory epithelium of the oral cavity and develops with a decrease (hyposialia) or complete absence of salivary secretion (asialia). Xerostomia caused by hypofunction of the salivary glands is a common problem among older people. This condition causes significant oropharyngeal disturbances, pain and impairs quality of life. The prevalence of xerostomia varies among the general population. Research shows that its frequency increases with age. Xerostomia is present in various diseases (bronchial asthma, rheumatoid arthritis, Parkinson's disease, diabetes mellitus, autoimmune thyroid diseases, including Graves disease and Hashimoto thyroiditis, systemic lupus erythematosus and many others) and upon prescription of more than 400 types of medications, whereas 100% patients with Sjögren's syndrome and patients who received radiation therapy (cancer of the head and neck) have xerostomia [14].

Dry mouth has many causes, from localized salivary problems to a variety of medications and medical conditions. Traditional treatment is aimed at eliminating the underlying cause and/or increasing salivary flow using local and systemic stimulants. Early intervention for dry mouth can prevent the harmful effects of this disorder. Xerostomia in many cases results in the growth of bacteria, an unpleasant odor, the appearance of ulcers, pain, swelling of tissues, i.e. inflammation. Glossitis (on the tongue), gingivitis (on the gums), cheilitis (on the lip) and stomatitis (on different parts of the oral mucosa) can develop depending on location of the infection. [15, 16]. Dry mouth occurs when the salivation rate at rest is less than the rate of fluid loss from the mouth, either through evaporation or absorption of water through the oral mucosa. By definition, evaporation can only occur during mouth breathing, and it is estimated that its maximum rate can be as high as 0.21 ml/min, although normally even in a mouth-breathing patient it is significantly less [17]. Symptoms of dry mouth may be caused by localized areas of mucosal dryness, particularly on the palate. To prevent this condition, an unstimulated salivary flow of >0.1-0.3 ml/min may be required [18] due to stimulation of the oral mucosa and secretory epithelium.

Currently, there is no targeted and epigenetic therapy for various human diseases mediated by a genetic or epigenetic decrease in the quantity of aquaporins 3 and 5 in body cells, therefore the authors proposed a beta-caryophyllene-based composition as a new aquaporin modulator.

Beta(β)-caryophyllene, known as trans-(1R,9S)-8-methylene-4,11,1, is found in parts of such common plant as cinnamon (Cinnamomum spp.), basil (Ocimum spp.), pepper (Piper spp.), breakfast mint (*Perilla frutescens* (L.) Britton), coriander (*Coriandrum sativum* L.), chestnut (*Aesculus hippocastanum* L.), sage (*Salvia officinalis* L.), cubeba pepper (*Piper cubeba* L.f.), thyme (*Thymus vulgaris* L.), myrrh [*Myrrhis odorata* (L.) Scop. ], curry leaves [*Murraya koenigii* (L.) Spreng.], hops (*Humulus lupulus* L.), cloves [*Syzygium aromaticum* (L.) Merr. & L.M. Perry], hemp (*Cannabis sativa* L.), lavender (*Lavandula angustifolia* Mill.), oregano (*Origanum vulgare* L.), rosemary (*Rosmarinus officinalis* L.), etc. Beta-caryophyllene is unique in terms of its wide dietary availability and occurrence in numerous plant genera and species [40]. β-Caryophyllene is primarily synthesized by plants as a defense mechanism against insects and aphids, and also plays a role in pollination.

This component has a number of important pharmacological properties, including antioxidant, anti-inflammatory, anticancer, cardioprotective, hepatoprotective, gastroprotective, nephroprotective, antimicrobial and immunomodulatory activities [41]. Beta-caryophyllene also performs an important function in the complex endocannabinoid system of the body. Beta-caryophyllene binds to CB2 receptors and improves overall health and well-being. These receptors work with enzymes and help maintain normal functioning of the nervous and immune systems. However, the specific effect of beta-caryophyllene on the stimulation of various types of aquaporins has not previously been identified. The authors found out unexpectedly that beta-caryophyllene affects the epigenetic activation and stimulation of AQP3 and AQP5 in the mucosal epithelium and keratinocytes lining various body cavities and organs, preventing xerosis, xerostomia and having a potential impact on the course of human diseases, such as hereditary palmoplantar keratoderma, hidradenitis supurativa, psoriasis, vitiligo, Alzheimer's disease and Sjögren's syndrome.

The prior art discloses DENTAID Xeros mouthwash to eliminate dry mucous membranes (https://doctorslon.ru/catalog/penki-i-opolaskivateli/opolaskivatel-dentaid-xeros-dlya-ustraneniya-sukhosti-500-ml/) containing the following components: betaine, potassium chloride, water, sodium fluoride, allantoin, hydroxyethylcellulose, glycerin, hydrogenated castor oil, xylitol, methylparaben, sodium chloride, propylene glycol, propylparaben, menthone glycerin acetal, sodium dihydrogen phosphate, sodium saccharin, sodium phosphate, flavoring. Betaine and xylitol are active ingredients in this product. However, betaine helps attract and retain water, but it does not address the underlying cause of dryness; xylitol is used as a means to prevent caries, but it does not affect salivation in any way. DENTAID® Xeros gel contains similar active substances to eliminate dry mouth (https://egigiena.ru/kupi/product/dentaid-xeros-gel-gel-dlya-ustraneniya-suhosti-polosti-rta-50-ml/), and additionally contains malic acid, which can stimulate salivation, but at the same time can cause demineralization of the enamel.

The prior art discloses Japanese moisturizing gel toothpaste NIPPON ZETTOC with fluoride QUOM for the care of the sick and elderly, which has the following composition: water, glycerin, sorbitol, silica, Gluconic Acid, Hydroxyethylcellulose, PEG-60 Hydrogenated Castor Oil, Sodium Hydroxide, Stannous Fluoride, Hakka Yu, Propylene Glycol Alginate, Dipotassium Glycyrrhizate, Pyridoxine HCL, Sodium Fluoride, Benzalkonium Chloride, Hydrolyzed Hyaluronic Acid. The active component here is hyaluronic acid, which can retain moisture only at the surface level, but does not solve the problem of insufficient aquaporins in the mucous membrane and epidermis cells. However, this component will not be effective for problems with the salivary glands and saliva production.

The prior art discloses Waterdent mouth moisturizing spray (https://www.asna.ru/cards/voterdent sprey_dpolosti_rta_uvlazhnyayushchiy_15ml_zelenaya _dubrava.html?utm_referrer=https%3A%2F%2Fwww.google.com%2F), which has the following composition: Aqua, Sorbitol, Glycerin, Paeonia Extract, Betaine, PEG-40 Hydrogenated Castor Oil, Panthenol, Sodium Benzoate, Sodium Hyaluronate, Potassium Sorbate, Aroma, o-Cymen-5-ol, Sodium Saccharin, Citric Acid. Panthenol, which is included in the composition, is able to fight the consequence of dryness, rather than the cause, since it functions as a softening component.

Glyceryl glucoside is often used as an active component in skin moisturizing products, which moisturizes the skin inside the epidermis due to the formation of hydrogen bonds with water. The action of glyceryl glucoside is aimed at binding and retaining water in the skin, while it penetrates the epidermis and reduces transepidermal water loss. For example, it is used in the product TRUE ALCHEMY VITAMIN C 2,0% + GLYCERYL GLUCOSIDE 0,9% (https://goldapple.ru/19000043213-vitamin-c-2-0-glyceryl-glucoside-0-9). Glyceryl glucoside is able to stimulate type 3 aquaporins, but this effect is insufficient according to experimental results.

Patent RU 2456977C1 [Demenko V.I., Shcherban V.P.] published on 27 June 2012 describes a cosmetic product for dry and very dry skin based on a ceramide complex and various cosmetic additives. The invention relates to aesthetic medicine, more precisely to cosmetology, and can be used for aging skin care. The invention contains a ceramide complex, an anti-irritant complex, a Langerhans cell activator, an hyaluronic acid synthesis activator, a natural moisturizing factor, as well as other cosmetically acceptable ingredients. The anti-irritation complex may contain aloe vera gel in combination with other plant extracts. The natural moisturizing factor may contain a mixture of betaine and other acids. The cosmetic product has high biological activity, increases and stabilizes the moisture content of the skin, and has a high lifting and rejuvenating effect.

The composition from patent RU 2456977C1 as described above is claimed by the authors to increase and stabilize the moisture content of the skin, has a complex multicomponent formula. The composition contains moisturizing agents glycerin and urea, occlusive agents from the group of silicones, a hyaluronic acid activator, and amino acid derivatives, however, no examples were given to show the effect of the composition on increasing the number of type 3 aquaporins in the epidermal cells of the skin. The composition may have any other mechanism of action due to the large number of cosmetically acceptable substances with known effects on skin cells. The main mechanism of action is creation of a kind of polylamellar layer on the keratin scale surface (degenerated keratinocytes), with a ceramide composition virtually corresponding to that in the skin, i.e. the composition is aimed at eliminating the consequences of low moisture content, but not eliminating the cause and influence on the natural processes inherent in epidermal cells. The effect on this mechanism of action helps strengthen the stratum corneum of the epidermis, but does not significantly increase moisture content, nor does it stimulate transepidermal aqua flow in cells or AQP3 formation to enhance the keratinocyte own cellular renewal, wound healing, and moisture delivery in all layers of the epidermis. The examples do not indicate how skin moisture content was measured in clinical trials, which limits correct interpretation of the data.

Patent RU 2643928C2 [LLC Pharmaceutical Reagents Institute Repharm] published on 6 February 2016 describes a medicinal and cosmetic product for external use related to the pharmaceutical industry. The product contains a composition of a mixture of disodium pyrophosphate and tetrapotassium pyrophosphate in a weight ratio of 1:1 and a bisphosphonate, lipids, emulsifiers, texturizers, biologically active and medicinal additives, flavorings, dyes, preservatives and water in a certain weight ratio of the mixture components (wt.%), described in the patent. Various compounds can be used as biologically active and medicinal additives, including natural betaine and aloe vera gel. The invention provides for reduced intensity of poorly soluble calcium salt crystallization in skin cells, thus eliminating the deposition of salts in the basal layer of the epidermis.

The composition from patent RU 2643928C2 as described above is claimed by the authors to reduce the crystallization of poorly soluble calcium salts, but has a complex multicomponent composition. The main active ingredients are potassium and sodium pyrophosphates, as well as bisphosphonate, which have a high affinity for calcium ions. The use of the composition helps regulate mineral cellular metabolism and slow down the aging process in skin cells; however, the use of the composition for moisturizing the skin, pH-dependent stimulation of the quantity of type 3 aquaporins in the skin and regulation of transepidermal aqua flow in skin cells has not been described. The effect on this mechanism of action helps normalize calcium metabolism, eliminate calcifications in the skin during the aging process, but does not significantly increase moisture content, nor does it stimulate transepidermal aqua flow in cells or AQP3 formation to enhance the keratinocyte own cellular renewal, wound healing, and moisture delivery in all layers of the epidermis. The examples do not indicate how skin moisture content was measured in clinical trials, which limits correct interpretation of the data. Additionally, the authors showed that the use of aloe vera extract was not sufficient to stimulate the quantity of AQP3 in keratinocytes.

In comparison with the patents described above, the authors of the present invention investigated compositions of shower gels, shampoos, mouth rinses, xerostomia prevention and treatment gels, and for the first time established effectiveness of beta-caryophyllene, epigenetic regulation and potential prevention of water-osmotic conditions of the mucous membranes and epithelium. The technical result of using beta-caryophyllene is to stimulate the quantity of AQP3 and AQP5 to maintain water-osmotic balance and simultaneous redistribution of transepidermal aquaflow in keratinocytes and epidermal cells. The component is active in the pH range of 5.0-8.0, which corresponds to most areas of the mucous membranes of the mouth, nose, eye (conjunctiva) and epidermis of the skin, scalp, and exocrine glands. The phytocomponent does not disrupt cellular structures throughout the exposure, affects the epigenetically embedded processes of types 3 and 5 aquaporin regulation in the cells themselves, does not form an occlusive film on the surface of cells, and facilitates water-osmotic exchange.

An additional property is decreasing the transepidermal water level (TEWL), dermatological tolerability and safety of use of the beta-caryophyllene containing products in volunteers and patients with various diseases. The use of the component helps ensure prevention and/or treatment of various diseases mediated by AQP3 and AQP5 dysfunction in various tissues, and improve the condition of the mucous membranes of the mouth, nose, eyes and skin. Additionally, beta-caryophyllene is 100% natural, biodegradable, and does not contain any components of animal origin.

SUMMARY OF THE INVENTION

In the first aspect, the invention relates to beta-caryophyllene for the care of body and scalp skin and mucous membranes of the mouth, nose, eyes and secretory glands, in particular for the pH-dependent regulation of aquaporins types 3 and 5, and water-osmotic balance regulation. The invention relates to beta-caryophyllene of plant origin from essential oils, fruits, seeds, leaves, grass and/or flowers, where the mass content of the component equivalent to the active substance is 0.01-0.3 wt.%.

The invention differs in that beta-caryophyllene (β-caryophyllene) is a substance with CAS registration number 87-44-5 or 10579-93-8. The beta-caryophyllene may be a plant-derived beta-caryophyllene such as *(+)-beta-Caryophyllene, (-)-beta-caryophyllene, Caryophyllene beta natural Ventos, Caryophyllene 917 indesso natural Ventos, Caryophyllene 924 indesso Ventos* or other commercially available raw materials.

Beta-caryophyllene may be a levorotatory beta-caryophyllene, a dextrorotatory beta-caryophyllene, a racemic beta-caryophyllene or a plant essential oil containing all forms of beta-caryophyllene.

The composition of the present invention may contain, wt.%:

| Beta-caryophyllene | 0.01-0.30%. |
|---|---|

In a second aspect, the invention relates to the use of beta-caryophyllene to stimulate the quantity of type 3 aquaporins and/or type 5 aquaporins in the mucous membranes of the mouth, nose, eyes, skin, secretory glands or epidermal layers of the skin and scalp.

In a third aspect, the invention relates to the use of the composition of the present invention for pH-dependent regulation of type 3 aquaporins and distribution of transepidermal moisture flow in the skin cells of the body and scalp, and oral mucous membranes.

In a fourth aspect, the invention relates to face and body care products, in particular lotions, creams, serums, cleansing gels, masks, mist sprays, gels, oleogels, balms, butters, containing 0.01-0.30 wt.% according to the present invention.

In a fifth aspect, the invention relates to oral care products, in particular toothpastes, dental gels, mouth rinses, moisturizing gels, oral serums, absorbable forms, solid powders containing 0.01-0.30 wt. % according to the invention.

In a sixth aspect, the invention relates to scalp care products, in particular scalp and hair care shampoos, shampoos for dandruff, seborrheic dermatitis and xerosis of the scalp, lotions, creams, serums, gels, cleansers containing 0.01 -0.30 wt.% according to the invention.

In a seventh aspect, the invention relates to face and body cleansers, such as cleansing gels, cleansing scrub gels, cleansing mousse foam, micellar water, liquid soap for the body, hands and face, solid soap, containing 0.01-0.30 wt.% according to the invention.

In the eighth aspect, the invention relates to products for the nasal mucosa care, in particular creams, lotions, balms, sticks, sprays, irrigations, gels, oleogels, containing 0.01-0.30 wt.% according to the invention.

In a ninth aspect, the invention relates to the use of beta-caryophyllene for the prevention and/or treatment of diseases mediated by type 3 aquaporins and/or type 5 aquaporins.

DETAILED DESCRIPTION OF THE INVENTION

In the first aspect, the invention relates to beta-caryophyllene for the care of body and scalp skin and mucous membranes of the mouth, nose, eyes and secretory glands, in particular for the pH-dependent regulation of aquaporins types 3 and 5, and water-osmotic balance regulation. The invention relates to beta-caryophyllene of plant origin from essential oils, fruits, seeds, leaves, grass and/or flowers, where the mass content of the component equivalent to the active substance is 0.01-0.3 wt.%.

The invention differs in that beta-caryophyllene (β-caryophyllene) is a substance with CAS registration number 87-44-5 or 10579-93-8. Beta-caryophyllene (INCI: β-caryophyllene) is a commercially available ingredient in the products.

Beta-caryophyllene may be a levorotatory beta-caryophyllene, a dextrorotatory beta-caryophyllene, a racemic beta-caryophyllene or a plant essential oil containing all forms of beta-caryophyllene. These molecules are well known to those skilled in the art and are commercially available from various suppliers.

The composition of the present invention may contain, wt.%:

| Beta-caryophyllen | 0.01-0.30%. |
|---|---|

In the composition of the invention, suitable auxiliary substances may be selected from the following categories of components.

Anionic surfactants:

1. Salts of higher carboxylic acids with the general formula R1-CO2X1, where R1 is an alkyl and/or alkenyl group with the hydrocarbon chain length from 5 to 21 carbon atoms, and X1 is a cation of alkali and/or alkali-earth metal, ammonium, alkylammonium, alkanolammonium, glucoammonium, basic amino acid;

2. Salt of a higher fatty acid amide and methylglycine with the general formula R4-C(O)-N(-CH3)-CH2-CO2X4, where R4 is an alkyl and/or alkenyl group with the hydrocarbon chain length from 5 to 21 carbon atoms, and X4 is a cation of alkali and/or alkali-earth metal, ammonium, alkylammonium, alkanolammonium, glucoammonium;

3. Alkyl polyethylene glycol carboxylate with the general formula R5-O(-CH2-CH2-O-)n2CH2-CO2X5, where n2 can take values from 1 to 15, and denotes the number of polyethylene glycol groups, R5 is an alkyl and/or alkenyl group with long hydrocarbon chain from 6 to 22 carbon atoms, and X5 is a cation of an alkali and/or alkali-earth metal, ammonium, alkylammonium, alkanolammonium, glucoammonium;

4. Alkyl sulfate with the general formula R3-OSO3X3, where R3 is an alkyl and/or alkenyl group with the hydrocarbon chain length from 6 to 22 carbon atoms, and X3 is a cation of alkali and/or alkali-earth metal, ammonium, alkylammonium, alkanolammonium, glucoammonium;

5. Alkyl polyethylene glycol sulfate with the general formula R2-O(-CH2-CH2-O)n1SO3X2, where n1 can take values from 1 to 10 and denotes the number of polyethylene glycol groups, R2 is an alkyl and/or alkenyl group with the hydrocarbon chain length from 6 to 22 carbon atoms, and X2 is a cation of alkali and/or alkali-earth metal, ammonium, alkylammonium, alkanolammonium, glucoammonium;

6. Disubstituted salt of 2-sulfocarboxylic acid with the general formula R6-CH(-SO3X6)-CO2X6, where R6 is an alkyl and/or alkenyl group with the hydrocarbon chain length from 4 to 20 carbon atoms, and X6 is a cation of alkali and/or alkali-earth metal, ammonium, alkylammonium, alkanolammonia, glucoammonium;

7. Mono or disubstituted salt of the amide of a higher carboxylic acid and glutamic acid with the general formula R7-C(O)-NH-CH(-CH2-CH2-CO2X7)-CO2X7, where R7 is an alkyl and/or alkenyl group with the hydrocarbon chain length from 5 to 21 carbon atoms, and X7 is a cation of an alkali and/or alkali-earth metal, ammonium, alkyl ammonium, alkanol ammonium, glucoammonium or hydrogen;

8. Salt of a higher fatty acid amide and glycine with the general formula R8-C(O)-NH-CH2-CO2X8, where R8 is an alkyl and/or alkenyl group with the hydrocarbon chain length from 5 to 21 carbon atoms, and X8 is a cation of alkali and/or alkali-earth metal, ammonium, alkylammonium, alkanolammonium, glucoammonium;

9. Salt of a higher fatty acid amide and alanine with the general formula R9-C(O)-NH-CH(-CH3)-CO2X9, where R9 is an alkyl and/or alkenyl group with the hydrocarbon chain length from 5 to 21 carbon atoms, and X9 is a cation of an alkali and/or alkali-earth metal, ammonium, alkylammonium, alkanolammonium, glucoammonium;

10. Salt of a higher fatty acid amide and 2-aminomethylethanesulfonic acid with the general formula R10-C(O)-N(-CH3)-CH2-CH2-SO3X10, where R10 is an alkyl and/or alkenyl group with the hydrocarbon chain length from 5 to 21 carbon atoms, and X10 is a cation of an alkali and/or alkali-earth metal, ammonium, alkylammonium, alkanolammonium, glucoammonium;

11. Alkyl polyglucoside hydroxypropyl sulfonate with the general formula: R11-O-[G]p1-O-CH2-CH(-OH)-CH2-SO3X11, where R11 is an alkyl and/or alkenyl group with the hydrocarbon chain length from 6 to 22 carbon atoms, G is a saccharide fragment containing 5 or 6 carbon atoms, p1 can take values from 1 to 4, and X11 is a cation of an alkali and/or alkali-earth metal, ammonium, alkylammonium, alkanolammonium, glucoammonium;

12. Alkyl polyglucoside carboxylate with the general formula R12-O-[G]p2-O-CH2-CO2X12, where R12 is an alkyl and/or alkenyl group with the hydrocarbon chain length from 6 to 22 carbon atoms, G is a saccharide fragment containing 5 or 6 carbon atoms, p2 can take values from 1 to 4, and X12 is a cation of an alkali and/or alkali-earth metal, ammonium, alkylammonium, alkanolammonium, glucoammonium;

13. Salt of the amide of a higher fatty acid and threonine with the general formula R13-C(O)-NH-CH(-CH(-OH)-CH3)-CO2X13, where R13 is an alkyl and/or alkenyl group with the hydrocarbon chain length from 5 to 21 carbon atoms, and X13 is a cation of an alkali and/or alkali-earth metal, ammonium, alkylammonium, alkanolammonium, glucoammonium;

14. Salt of a higher fatty acid amide and amino acid obtained by hydrolysis of proteins from plant materials, with the general formula R14-C(O)-AAX14, where R14 is an alkyl and/or alkenyl group with the hydrocarbon chain length from 5 to 21 carbon atoms, AA is an amino acid or peptide obtained by hydrolysis of vegetable protein (possible sources of protein are apple, soybean, wheat, cotton, etc.), and X14 is a cation of an alkali and/or alkali-earth metal, ammonium, alkylammonium, alkanolammonium, glucoammonium.

15. Rhamnolipids, which are glycolipid surfactants of microbial origin. The rhamnose units can be present as either mono- (one) or di- (two) units linked by α-1,2-glycosidic bonds. The lipid units are linked by ester bonds between β-hydroxy groups, can be saturated, monounsaturated or polyunsaturated and can have chain lengths from C8 to C16.

Amphoteric surfactants:

1. Disubstituted salt of acylamphodiacetate with the general formula R15-C(O)-NH-CH2-CH2-N(-CH2-CO2X15)-CH2-CH2-O-CH2-CO2X15, where R15 is an alkyl and/or alkenyl group with the hydrocarbon chain length from 5 to 21 carbon atoms, and X15 is a cation of an alkali and/or alkali-earth metal, ammonium, alkylammonium, alkanolammonium, glucoammonium;

2. Acyl amphoacetate salt with the general formula R16-C(O)-NH-CH2-CH2-N(-CH2-CO2X16)-CH2-CH2-OH, where R16 is an alkyl and/or alkenyl group with the hydrocarbon chain length from 5 to 21 carbon atoms, and X16 is a cation of an alkali and/or alkali-earth metal, ammonium, alkylammonium, alkanolammonium, glucoammonium;

3. Alkylamphoacetate salt with the general formula R17-C(=N-CH2-CH2-N((-CH2-CH2-OH)-CH2-CO2X17)-), where R17 is an alkyl and/or alkenyl group with the hydrocarbon chain length from 5 to 21 carbon atoms, and X17 is a cation of an alkali and/or alkali-earth metal, ammonium, alkylammonium, alkanolammonium, glucoammonium;

4. Acylamidoalkylbetaine with the general formula R18-C(O)-NH-R19-N(-CH3)2)-CH2-CO2, where R18 is an alkyl and/or alkenyl group with the hydrocarbon chain length from 5 to 21 carbon atoms, and R19 is an alkyl group with the hydrocarbon chain length from 1 to 4 carbon atoms;

5. Acylamidoalkylhydroxysultaine with the general formula R20-C(O)-NH-R21-N(-CH3)2-CH2-CH(-OH)-CH2-SO3, where R20 is an alkyl and/or alkenyl group with the hydrocarbon chain length from 5 to 21 carbon atoms, and R21 is an alkyl group with the hydrocarbon chain length from 1 to 4 carbon atoms;

6. Acylamidoalkylamine oxide with the general formula R22-C(O)-NH-R23-N(-CH3)2-O, where R22 is an alkyl and/or alkenyl group with the hydrocarbon chain length from 5 to 21 carbon atoms, and R23 is an alkyl group with the hydrocarbon chain length from 1 to 4 carbon atoms;

7. Alkylbetaine with the general formula R24-N(-CH3)2)-CH2-CO2, where R24 is an alkyl and/or alkenyl group with the hydrocarbon chain length from 5 to 21 carbon atoms;

8. Alkylhydroxysultaine with the general formula R25-N(-CH3)2-CH2-CH(-OH)-CH2-SO3, where R25 is an alkyl and/or alkenyl group with the hydrocarbon chain length from 6 to 22 carbon atoms;

9. Alkylsultaine with the general formula R26-N(-CH3)2-CH2-CH2-CH2-SO3, where R26 is an alkyl and/or alkenyl group with the hydrocarbon chain length from 6 to 22 carbon atoms;

10. Alkylamine oxide with the general formula R27-N(-CH3)2-O, where R26 is an alkyl and/or alkenyl group with the hydrocarbon chain length from 6 to 22 carbon atoms.

Nonionic surfactants:

1. Alkylpolyethylene glycol with the general formula R29-O(-CH2-CH2-O-)n3H, where n3 can take values from 2 to 20 and denotes the number of polyethylene glycol groups, and R29 is an alkyl and/or alkenyl group with the hydrocarbon chain length from 6 to 22 carbon atoms;

2. Alkylpolyethylene/propylene glycol with the general formula R30-O(-CH2-CH2-O-)n4(-CH(-CH3)-CH2-O-)n5H, where n4 can take values from 2 to 20 and denotes the quantity polyethylene glycol groups, n5 can take values from 2 to 20 and denotes the number of polypropylene glycol groups, and R30 is an alkyl and/or alkenyl group with the hydrocarbon chain length from 6 to 22 carbon atoms;

3. Dialkylpolyethylene glycol with the general formula R31-O(-CH2-CH2-O-)n6R32, where n6 can take values from 2 to 20 and denotes the number of polyethylene glycol groups, R31 is an alkyl and/or alkenyl group with the hydrocarbon chain length from 6 to 22 carbon atoms, and R32 is an alkyl and/or alkenyl group with the hydrocarbon chain length from 1 to 12 carbon atoms;

4. Dialkylpolyethylene/propylene glycol with the general formula R33-O(-CH2-CH2-O-)n7(-CH(-CH3)-CH2-O-)n8-R34, where n7 can take values from 2 to 20 and denotes the number of polyethylene glycol groups, n8 can take values from 2 to 20 and denotes the number of polypropylene glycol groups, R33 is an alkyl and/or alkenyl group with the hydrocarbon chain length from 6 to 22 carbon atoms, and R34 is an alkyl and/or alkenyl group with the hydrocarbon chain length from 1 to 12 carbon atoms.

Disperse medium and solvent:

1. Organic alcohol with the general formula R35(-OH)s1, where R35 is an alkyl group with the hydrocarbon chain length from 3 to 12 carbon atoms, and S1 can take values from 1 to 12 and denotes the number of hydroxyl groups located in the hydrocarbon radical in any order relative to each other;
2. Alkylpolypropylene glycol with the general formula H(-CH(-CH3)-CH2-O-)n9R36, where n9 can take values from 2 to 10 and denotes the number of polypropylene glycol groups, and R36 is an alkyl group with the hydrocarbon chain length from 1 to 10 carbon atoms.

pH regulators:

1. Organic acids with the general formula R37(-OH)s2(-COOH)m1, where R37 is an alkyl group with the hydrocarbon chain length from 1 to 12 carbon atoms, S2 can take values from 1 to 12 and denotes the number of hydroxyl groups in the hydrocarbon radical, in random order relative to each other, and M1 can take values from 1 to 4 and denotes the number of carboxyl groups in the hydrocarbon radical, in random order relative to each other;
2. Solutions of hydroxides of alkali or alkali-earth metals, ammonia, primary and tertiary alkylamines, primary and tertiary alkanolamines, primary and tertiary glucamines, basic amino acids, disodium citric acid, trisodium citric acid.

Abrasive components:

1. Solid water-insoluble particles, which can be silicon dioxide with the formula $SiO_2$, calcium carbonate with the formula $CaCO_3$, various phosphorus-calcium salts, which are calcium pyrophosphate, dicalcium phosphate dihydrate, calcium hydroxyapatite, calcium orthophosphate, sodium tripolyphosphate.

Bleaching components:

2. Water-soluble calcium and phosphorus salts, which may be potassium tetrapyrophosphate, disodium pyrophosphate, sodium triphosphate, potassium tetrapyrophosphate, sodium tetrapyrophosphate, sodium hexametaphosphate, sodium tripolyphosphate.
3. Proteolytic enzymes, which can be plant proteases such as papain, bromelain, ficin, actinidin, as well as proteases isolated from microbiological sources, for example, subtilisin, bacillolysin.

Thickeners:

1. Polysaccharides, such as carboxymethylcellulose, carrageenan, xanth gum.

Sweeteners and sugar substitutes:

1. Synthetic and natural sweeteners and sugar substitutes, such as sucralose, xylitol, sodium saccharinate, stevia extract, erythritol, maltitol, sorbitol.

Chelating agents:

1. Trisodium salt of methylglycine diacetic acid, tetrasodium salt of glutamine diacetic acid, trisodium salt of ethylenediamine-(N,N)-disuccinate;

2. Organic acids, as well as salts of alkali metals, ammonium, alkylammonium, alkanolammonium, glucoammonium, corresponding to the following acids: citric acid, malic acid, tartaric acid, glutaric acid, adipic acid, glucuronic acid, galacturonic acid, galactaric acid, gluconic acid, phytic acid, polytaconic acid, polyacrylic acid, polymethacrylic acid, copolymer of acrylic and maleic acids, as well as organic acids with the general formula $R38(-OH)s3(-COOH)m2$, where R38 is an alkyl group with the hydrocarbon chain length from 1 to 12 carbon atoms, S3 - can take values from 1 to 12 and denotes the number of hydroxyl groups in the hydrocarbon radical, in random order relative to each other, and M2 can take values from 1 to 4 and denotes the number of carboxyl groups in hydrocarbon radical, in random order relative to each other.

Preservatives:

1. Organic acids and salts of alkali and alkali-earth metals, ammonium, alkylammonium, alkanolammonium, glucoammonium, corresponding to the following acids: benzoic acid, sorbic acid, 4-methoxybenzoic acid, salicylic acid, undecylenic acid;

2. Organic alcohols and phenols: phenoxyethanol, benzyl alcohol, caprylyl glycol, ethylhexylglycerin, phenethyl alcohol, 3-methyl-4-isopropylphenol, 2,4-dichlorobenzyl alcohol;

3. Broad-spectrum biocides: benzisothiazolinone, methylisothiazolinone, dodecyldipropylene triamine;

4. Fungicides: sodium pyrithione, climbazole.

Fragrances with or without the addition of essential oils of any olfactory direction for improved consumer properties.

Essential oils in pure form or in the form of mixtures in different proportions: orange, bergamot, lemon, lime, tangerine, grapefruit, neroli, rosewood, yuzu, lemongrass, lavender, sage, thyme, lemon balm, mint of various botanical types, tea tree, eucalyptus, cedarwood, sandalwood, black pepper, pink pepper, cinnamon, cardamom, coriander, jasmine, rose, peony, blue chamomile, basil, geranium, as well as any other commercially available essential oils known to those skilled in the art.

MEASUREMENT DATA

The examples included in this description are not limiting of the claimed invention and are provided for the purpose of illustration and confirmation of the expected technical result achievement only. These examples are among many experimental data obtained by authors of the invention that confirm effectiveness of the agents within the scope of the invention.

**Example 1.**

The components to be included in the composition of the present invention were studied within various perfumes and cosmetics. A liquid product for body skin cleansing, in particular a shower gel, was prepared under the scope of the present invention (Table No. 1).

Table No. 1. Ingredients of a shower gel with the claimed composition

| Item No. | Component | Content,% wt. |
|---|---|---|
| 1 | Purified water | up to 100 |
| 2 | Sodium coco sulfate | 3.5-10.0 |
| 3 | Decyl glucoside | 3.0-10.0 |
| 4 | Cocamidopropyl betaine 40-45% | 5.0-10.0 |
| 5 | Glutamate diacetate tetrasodium salt | 0.05-0.4 |
| 6 | Sodium chloride | 0.1-0.4 |
| 7 | Citric acid monohydrate | 0.1-0.5 |
| 8 | Potassium sorbate, sodium benzoate | 0.2-1.0 |
| 9 | Beta-caryophyllene | 0.01-0.2 |

(continued)

| Item No. | Component | Content,% wt. |
|----------|-----------|---------------|
| 10 | Trimethylglycine | 0.05-5.0 |
| 11 | *Aloe Barbadensis* Leaf Extract | 0.00005-0.1 |
| 12 | Fragrance with ingredients of natural origin | 0.0-0.8 |
| 13 | Essential oil or combination of essential oils | 0.0-0.8 |
| 14 | Aqueous solution of citric acid and silver citrate | 0.0-0.2 |

The prepared shower gel ensures high cleaning effect, foaming and foam stability, has a hypoallergenic effect, regulates pH of the body skin, increases the quantity of type 3 and 5 aquaporins, promotes the distribution of transepidermal aqua flow in the body skin cells and moisturizes the body skin due to beta-caryophyllene content. The use of shower gel with beta-caryophyllene helps specifically regulate the water-osmotic flow in the skin epithelium, prevent and alleviate skin xerosis, atopic dermatitis, contact dermatitis, eczema, psoriasis, and vitiligo.

**Example 2.**

The components to be included in the composition of the present invention were studied within various perfumes and cosmetics. A liquid product for scalp cleansing, in particular a scalp shampoo, was prepared under the scope of the present invention (Table No. 2)

Table No. 2. Ingredients of a scalp care shampoo with the claimed composition

| Item No. | Component | Content,% wt. |
|----------|-----------|---------------|
| 1 | Purified water | up to 100 |
| 2 | Sodium lauroyl methyl isothionate | 5.0-10.0 |
| 3 | Lauryl glucoside | 2.0-8.0 |
| 4 | Cocamidopropyl betaine 40-45% | 3.0-6.0 |
| 5 | Sodium cocoylisothionate | 1.0-4.0 |
| 6 | Glutamate diacetate tetrasodium salt 47% solution | 0.1-0.8 |
| 7 | Glycerol | 0.1-3.0 |
| 8 | Guar hydroxypropyltrimonium chloride | 0.1-0.5 |
| 9 | Citric acid monohydrate | 0.1-0.5 |
| 10 | Preservative | 0.2-1.0 |
| 11 | Beta-caryophyllene | 0.01-0.2 |
| 12 | *Trimethylglycine* | 0.05-5.0 |
| 13 | *Aloe Barbadensis Leaf Extract* | 0.001-0.1 |
| 14 | Fragrance | 0.05-0.5 |
| 15 | Essential oil or combination of essential oils | 0.05-0.6 |
| 16 | Additional active ingredients for hair care | 0.1-5.0 |

The prepared scalp and hair care shampoo ensures high cleaning effect, foaming and foam stability, has a hypoallergenic effect, a sebum-regulating effect, contributes to thickening of the hair shaft, increases its strength, normalizes hair porosity, moisturizes the hair, protects against thermal damage, regulates pH of the scalp, increases the quantity of type 3 aquaporins, promotes the distribution of transepidermal aqua flow in the body skin and scalp cells, moisturizes the body skin and scalp.

The prepared scalp care shampoo ensures high cleaning effect, foaming and foam stability, has a hypoallergenic effect, a sebum-regulating effect, regulates scalp pH and increases the quantity of 3 and 5 type aquaporins, promotes the distribution of transepidermal aqua flow in the scalp cells thanks to beta-caryophyllene content. The use of a scalp care shampoo with beta-caryophyllene helps specifically regulate the water-osmotic flow in the skin epithelium, prevent and alleviate scalp xerosis, dermatitis, psoriasis, seborrheic dermatitis and dandruff.

**Example 3.**

The components to be included in the composition of the present invention were studied within various perfumes and cosmetics. A liquid product for hand and body skin care, in particular liquid soap for hand and body washing, including the same for children and newborns, was prepared under the scope of the present invention (Table No. 3).

Table No. 3. Ingredients of a hand and body liquid soap with the claimed composition

| Item No. | Component | Content,% wt. |
|---|---|---|
| 1 | Purified water | up to 100 |
| 2 | Glycerol | 0.1-3.0 |
| 3 | Sodium coco sulfate | 3.5-10.0 |
| 4 | Coco glucoside | 3.0-10.0 |
| 5 | Sunflower oil methylglucamide | 0.1-5.0 |
| 6 | Cocamidopropyl betaine 40-45% | 2.0-7.0 |
| 7 | Glutamate diacetate tetrasodium salt 47% solution | 0.05-0.4 |
| 8 | Lactic acid | 0.2-0.9 |
| 9 | Preservative | 0.2-1.0 |
| 10 | Water-glycerin extract of cotton | 0.01-0.5 |
| 11 | Beta-caryophyllene | 0.01-0.2 |
| 12 | *Aloe Barbadensis* Leaf Extract | 0.00005-0.1 |
| 13 | Trimethylglycine | 0.0-5.0 |
| 14 | Fragrance | 0.0-0.8 |
| 15 | Aqueous solution of citric acid and silver citrate | 0.0-0.2 |
| 15 | Other additional ingredients | 0.005-1 |

The prepared hand and body liquid soap ensures high cleaning effect, foaming and foam stability, has a hypoallergenic effect, regulates body skin pH and increases the quantity of 3 and 5 type aquaporins, promotes the distribution of transepidermal aqua flow in the scalp cells thanks to beta-caryophyllene content. The use of liquid soap for hands and body with beta-caryophyllene helps specifically regulate the water-osmotic flow in the skin epithelium, prevent and alleviate skin xerosis, atopic dermatitis, contact dermatitis, eczema, psoriasis, and vitiligo.

**Example 4.**

The components to be included in the composition of the present invention were studied within various perfumes, cosmetics and pharmaceutical products. An emulsion for face, hand and body skin care, in particular a cream for the prevention and treatment of dermatological diseases, was prepared under the scope of the present invention (Table No. 4).

Table No. 4. Ingredients of a face, hand and body cream with the claimed composition

| Item No. | Component | Content,% wt. |
|---|---|---|
| 1 | Purified water | up to 100 |
| 2 | Glycerin | 0.1-3.0 |
| 3 | Xanthan Gum | 0.1-0.8 |
| 4 | Sodium Acrylate/Sodium Acryloyldimethyl Taurate Copolymer | 0-0.5 |
| 5 | Cetearyl Alcohol, Potassium Cetyl Phosphate | 0.5-6.0 |

(continued)

| Item No. | Component | Content,% wt. |
|---|---|---|
| 6 | Isopropyl Myristate | 0.5-6.0 |
| 7 | Glycine Soja (Soybean) Oil or Olea Europaea (Olive) Seed Oil or Prunus Persica (Peach) Kernel Oil or Prunus Amygdalus Dulcis (Sweet Almond) Oil or Butyrospermum parkii (Shea) Butter or Cocos Nucifera Oil or Mangifera Indica (Mango) Seed Butter or another plant oil/-butter | 0.5-5.0 |
| 8 | Beta-caryophyllene | 0.01-0.2 |
| 9 | Caprylic/Capric Triglyceride | 0.5-4.0 |
| 10 | Preservative | 0.2-1.0 |
| 11 | Chelating agent | 0.0-0.5 |
| 12 | Fragrance | 0.0-0.8 |
| 13 | Other additional ingredients | 0.005-3 |
| 14 | pH regulator | 0.01-0.5 |

The prepared face, hand and body cream provides a softening and regenerating effect, restores the skin lipid barrier, reduces keratinization of the skin, has a hypoallergenic effect, regulates pH of the hand skin, increases the number of type3 and 5 aquaporins, and promotes the distribution of transepidermal aqua flow in skin cells. The use of a lipid-replenishing cream with beta-caryophyllene helps specifically regulate the water-osmotic flow in the skin epithelium, prevent and alleviate skin xerosis, atopic dermatitis, contact dermatitis, eczema, psoriasis, and vitiligo.

**Example 5.**

The components to be included in the composition of the present invention were studied within various perfumes, cosmetic and pharmaceutical products. A therapeutic and prophylactic oral care product was prepared, in particular a foamy mouth rinse for the treatment of xerostomia, under the scope of the present invention (Table No. 5).

| Item No. | Component | Content,% wt. |
|---|---|---|
| 1 | Purified water | up to 100 |
| 2 | Glycerol | 1.0-10.0 |
| 3 | Sorbitol | 0.01-0.1. |
| 4 | Flavoring | 0.01-3.0 |
| 5 | Benzyl alcohol | 0.05 - 0.7 |
| 6 | Thickener | 0.5-5.0 |
| 7 | Protease | 0.5-5.0 |
| 8 | Beta-caryophyllene | 0.01-0.2 |
| 9 | Solubilizer | 0.005-0.50 |
| 10 | Other additional ingredients | 0.2-2.0 |
| 11 | pH regulator | 0.001-0.5 |
| 12 | Purified water | 0.005-3 |
| 13 | Glycerol | 0.001-0.5 |

The prepared mouth rinse stimulated salivation, had refreshing, antibacterial properties, and maintained a normal pH balance in saliva and dental plaque biofilm. The use of an oral product with beta-caryophyllene helps to effectively maintain oral hydration and prevent xerostomia.

**Example 6.**

The components to be included in the composition of the present invention were studied within various perfumes, cosmetic and pharmaceutical products. A therapeutic and prophylactic oral care product was prepared, in particular an oral cavity moisturizing gel for the treatment of xerostomia, under the scope of the present invention (Table No. 6).

| Item No. | Component | Content,% wt. |
|---|---|---|
| 1 | Purified water | up to 100 |
| 2 | Glycerol | 1.0-10.0 |
| 3 | Sorbitol | 1.0-10.0 |
| 4 | Flavoring | 0.01-3.0 |
| 5 | Benzyl alcohol | 0.05 - 0.7 |
| 6 | Thickener | 0.05-5.0 |
| 7 | Protease | 0.01-0.5 |
| 8 | Beta-caryophyllene | 0.01-0.2 |
| 10 | Solubilizer | 0.2-2.0 |
| 11 | Other additional ingredients | 0.005-3 |
| 12 | pH regulator | 0.001-0.5 |

The prepared oral gel ensured stimulation of salivation, effectively moisturized the oral mucosa tissues, had refreshing, antibacterial properties, and maintained a normal pH balance in saliva and dental plaque biofilm. The use of an oral product with beta-caryophyllene helps to effectively maintain oral hydration and prevent xerostomia.
Preclinical and clinical studies were conducted to evaluate the effectiveness and stability of the composition of the present invention in various perfumes and cosmetics according to the invention.

**Example 7.**

Beta-caryophyllene was studied for cytotoxicity and assessed for viability of skin and mucous membrane keratinocytes to determine the maximum permissible concentration for external and local application.
Cell viability was determined colorimetrically using MTT stain (Sigma Chemical, St. Louis, Mo.). The preparation was treated for the test in dimethylsulphoxide culture medium and added to a 96-well plate at serial dilutions ranging from 1.000 to 0.0032 mg/mL using a dilution factor of 3.16. Pre-culture was carried out for 48 hours before the test. Then MTT was added to the culture at a concentration of 0.75 mg/mL (100 $\mu$l/well) and incubated for another 4 hours. The contents of the well were removed, and 100 $\mu$l of isopropanol was added to solubilize the formed formazan crystals. Absorbance was determined in each well at 570 nm using a Multiskan GO monochromator (Thermo Scientific, Finland). Cell viability was expressed as a percentage and calculated by the equation:

$$\% \text{ Viable cells} = \frac{\text{Absorbance of evaluated product incubated cells}}{\text{Control absorbance}} \times 100$$

Three biological replicates were made. Statistical data processing was carried out to calculate the mean, standard deviation, and trend for each biological replicate. For the statistical evaluation ANOVA test was used allowing the measurement of variation of the results, comparing the data between all the groups. The Bonferroni post-test was then applied, which reinforced and made even more accurate the result presented in the ANOVA test. For both analyses, the significance level of 5% was used.

Results.

Based on the results of the initial cytotoxicity assessment, it was revealed that beta-caryophyllene does not cause death of skin keratinocytes in the claimed concentration range of 0.01-0.3 wt.%. The viability of skin and epithelial keratinocytes was maintained throughout the selected concentration range of the composition components and above the threshold value of 70%, which indicates the gentle effect of beta-caryophyllene on keratinocytes and its high activity (Fig.

1). Beta-caryophyllene showed noncytotoxic concentrations ranging from 0.0003 mg/mL to 0.3165 mg/mL, corresponding to 70% cell survival (EC70). EC70 was 0.3165 mg/ml.

**Example 8.**

An in vitro study was carried out to determine the quantity of type 3 aquaporins (AQP3) in keratinocytes of the skin epidermis after using the components of the composition according to the invention.

Aquaporins are proteins that form pores in cells to regulate transepidermal aqua flow and low molecular weight substances such as glycerol. Type 3 aquaporins (AQP3) are found mainly in skin cells, but also in cells of the respiratory system and kidneys to regulate aqua flow. The quantity of AQP3 in the basal and suprabasal layers directly determines the degree of water-osmotic balance, moisture penetration in various layers of the skin to the epidermis, normal differentiation of keratinocytes and their desquamation, as well as maintenance of the skin barrier layer.

The second step is to determine the quantity of AQP3 in skin keratinocytes after cultivation under standard conditions, using non-toxic concentrations of the component from previous testing: 0.010, 0.0032 and 0.001 mg/mL. A commercially available sandwich enzyme immunoassay test was used to determine the quantity of AQP3. HaCaT cells were added to a 96-well plate at a density of 105 cells per well. The next day, the cell medium was removed and replaced with 50 μL of fresh cell medium DMEM + 5% FBS to maintain cell growth. Then 50 μL samples of the composition according to the present invention were added and incubated for 48 hours. Cultivation conditions: humidified atmosphere with 5% $CO_2$ at 37°C. After the incubation, the cell supernatant was collected, the quantity of type 3 aquaporins in skin keratinocytes was then determined by ELISA method. Biotin-conjugated specific antibodies were bound to AQP3, and avidin-conjugated horseradish peroxidase was then added to bind the antibodies and change color with TMB. Only after binding of all 3 components of the reagent together with AQP3 was it possible to obtain a stable color, which was fixed with sulfuric acid and detected monochromatically at a wavelength of 450 + 10 nm using Multiskan GO. Purified water was used as a negative control while aloe vera extract, a mixture of glycerin and glyceryl glucoside, known for its ability to influence the AQP3 quantity in skin cells, was used as a positive control.

Three biological replicates were made. Statistical data processing was carried out to calculate the mean, standard deviation, and trend for each biological replicate. For the statistical evaluation ANOVA test was used allowing the measurement of variation of the results, comparing the data between all the groups. The Bonferroni post-test was then applied, which reinforced and made even more accurate the result presented in the ANOVA test. For both analyses, the significance level of 5% was used.

Results.

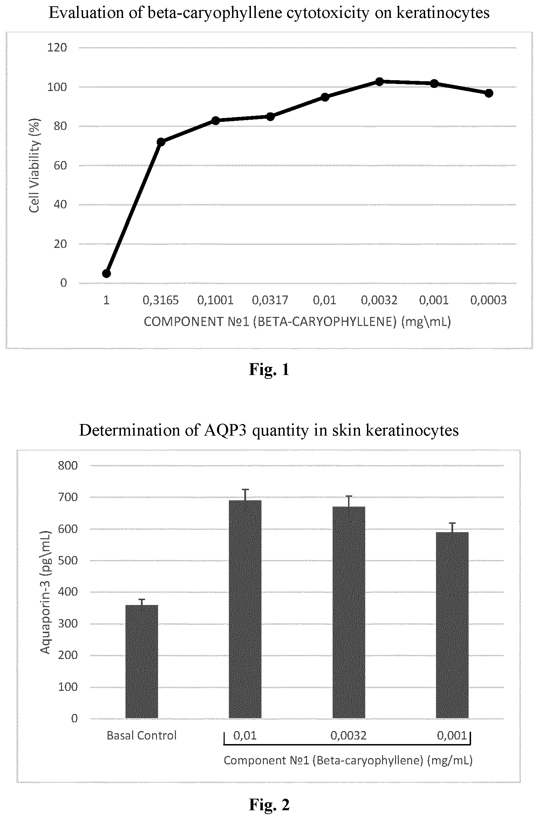

Based on the results of AQP3 quantity determination in skin keratinocytes after adding the components of the composition to the culture medium, activity of beta-caryophyllene was detected in small concentrations from 0.03 to 0.25 wt.% (Fig. 2), which indicates the highly active properties of beta-caryophyllene. The effect of *Aloe Barbadensis* leaf extract and glyceryl glucoside showed a slight increase in the quantity of AQP3 as compared to the negative control (Table No. 7). The data obtained show a long-term improvement in transepidermal aqua flow in keratinocytes and epigenetic regulation of the AQP3 quantity for the prevention of dermatological diseases and restoration of the skin water-osmotic balance.

Table No. 7. Estimation of the AQP3 quantity in skin keratinocytes in the presence of substances

| Test sample | Components of the composition | AQP3 quantity increase as compared to negative control, % |
|---|---|---|
| No. 1 | negative control (purified water) | - |
| No. 2 | *Aloe Barbadensis* Leaf Extract 0.01 wt.% equivalent to the active substance | +20.61% |
| No. 3 | Positive control (glycerol and glyceryl glucoside), 1 wt.% on raw material basis | +23.48% |
| No. 4 | Beta-caryophyllene, 0.08 wt.% | +92.87% |

Pre-treatment of epidermal cell culture (10-15 layers) with beta-caryophyllene is able to epigenetically regulate and increase the quantity of AQP3 by 65.46% (p<0.05), 92.87% (p<0.01) and 99.97% (p<0.01) at estimated concentrations of 0.001, 0.0032 and 0.010 mg/mL (corresponding to 0.03%, 0.08% and 0.25 wt%, respectively) as compared to the negative/basal control. The data are given as mean $\pm$ standard deviation of 3 biological replicates (ANOVA, Bonferroni).

Aquaporins type 3 (AQP3) in the basal and suprabasal layers of the skin epidermis enable water-osmotic metabolism regulation due to their own stimulation of cell penetration by low-molecular substances, in particular water. A fundamentally new, but natural mechanism ensures deep penetration of the aqua flow in all layers of the epidermis and in the initial dermis layer. AQP3 is very sensitive to the product pH, heavy metals, UVA and UVB radiation, epigenetic factors, and performs important function in the development of dermatological diseases, in particular atopic dermatitis, eczema, psoriasis, vitiligo, and skin xerosis.

Natural beta-caryophyllene is an innovative substance for targeted and epigenetic stimulation of the AQP3 quantity in epidermal keratinocytes and the incorporation of aquaporins into cell membranes for the aqua flow to enter. An increase in water content in skin cells stimulates their metabolism, renewal and normal maturation of keratinocytes, and ensures regeneration. The component helps reduce TEWL, increase the quantity of AQP3 in the epidermal cells of the mucous membranes and skin for the long-term prevention of xerosis caused by a decrease in the AQP3 quantity due to genetic features of the body. Unlike analogues, the activity of beta-caryophyllene in low concentrations makes it possible to epigenetically stimulate the synthesis of AQP3, ensuring deep transepidermal aqua flow into all epidermal cells and reducing skin xerosis. Deep hydration means an increase in transepidermal aqua flow in all 5 layers of the epidermis where AQP3 are located. The component affects the genetically inherent hydration processes in the skin itself and does not form an occlusive film on the cells surface, as confirmed by the positive results of the skin keratinocyte cytotoxicity study. This application would help prevent and correct dermatological diseases such as atopic dermatitis in newborns, children and adults, psoriasis, eczema, contact dermatitis, hyperkeratosis, epidermal hyperplasia, mast cell activation and allergic conditions.

**Example 9.**

An in vitro study was carried out to determine the quantity of type 5 aquaporins (AQP5) in keratinocytes of the skin epidermis after using the components of the composition according to the invention.

Aquaporins are proteins that form pores in cells to regulate transepidermal aqua flow and low molecular weight substances such as glycerol. Aquaporins type 5 (AQP5) are found mainly in skin cells, but also in cells of the respiratory system and kidneys to regulate aqua flow. The quantity of AQP5 in the basal and suprabasal layers directly determines the degree of water-osmotic balance, moisture penetration in various layers of the skin to the epidermis, normal differentiation of keratinocytes and their desquamation, as well as maintenance of the skin barrier layer.

The second step is to determine the quantity of AQP5 in skin keratinocytes after cultivation under standard conditions, using non-toxic concentrations of the component from previous testing: 0.010, 0.0032 and 0.001 mg/mL. A commercially available sandwich enzyme immunoassay test was used to determine the quantity of AQP5. HaCaT cells were added to a 96-well plate at a density of 105 cells per well. The next day, the cell medium was removed and replaced with 50 $\mu$L of fresh cell medium DMEM + 5% FBS to maintain cell growth. Then 50 $\mu$L samples of the composition according to the present invention were added and incubated for 48 hours. Cultivation conditions: humidified atmosphere with 5% $CO_2$ at 37°C. After the incubation, the cell supernatant was collected, the quantity of type 5 aquaporins in skin keratinocytes was then determined by ELISA method. Biotin-conjugated specific antibodies were bound to AQP5, and avidin-conjugated horseradish peroxidase was then added to bind the antibodies and change color with TMB. Only after binding of all 3 components of the reagent together with AQP5 was it possible to obtain a stable color, which was fixed with sulfuric acid and detected monochromatically at a wavelength of 450 + 10 nm using Multiskan GO. Purified water was used as a negative control while aloe vera extract, a mixture of glycerin and glyceryl glucoside, known for its ability to influence the AQP5 quantity in skin cells, was used as a positive control.

Three biological replicates were made. Statistical data processing was carried out to calculate the mean, standard deviation, and trend for each biological replicate. For the statistical evaluation ANOVA test was used allowing the measurement of variation of the results, comparing the data between all the groups. The Bonferroni post-test was then applied, which reinforced and made even more accurate the result presented in the ANOVA test. For both analyses, the significance level of 5% was used.

Results.

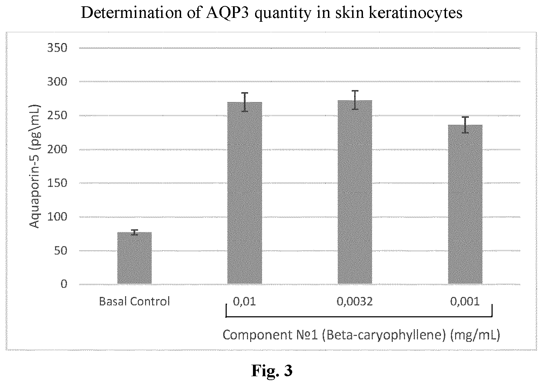

Based on the results of AQP5 quantity determination in skin keratinocytes after adding the components of the composition to the culture medium, activity of beta-caryophyllene was detected in small concentrations from 0.03 to 0.25 wt.% (Fig. 3), which indicates the highly active properties of beta-caryophyllene. The effect of *Aloe Barbadensis* leaf extract and glyceryl glucoside showed a slight increase in the quantity of AQP5 as compared to the negative control (Table No. 8). The data obtained show a long-term improvement in transepidermal aqua flow in keratinocytes and epigenetic regulation of the AQP5 quantity for the prevention of dermatological diseases and restoration of the skin water-osmotic balance.

Table No. 8. Estimation of the AQP5 quantity in skin keratinocytes in the presence of substances

| Test sample | Components of the composition | AQP5 quantity increase as compared to negative control, % |
|---|---|---|
| No. 1 | negative control (purified water) | - |
| No. 2 | *Aloe Barbadensis* Leaf Extract 0.01 wt.% equivalent to the active substance | +20.61% |
| No. 3 | Positive control (glycerol and glyceryl glucoside), 1 wt.% on raw material basis | +23.48% |
| No. 4 | Beta-caryophyllene, 0.08 wt.% | +92.87% |

Pre-treatment of epidermal cell culture (10-15 layers) with beta-caryophyllene is able to epigenetically regulate and increase the quantity of AQP5 by 332.26% ($P<0.01$), 335.77% ($P<0.01$) and 264.89% ($P<0.05$) at estimated concentrations of 0.001, 0.0032 and 0.010 mg/ml (corresponding to 0.25%, 0.08% and 0.03 wt%, respectively) relative to the negative/base control. Data represent the mean $\pm$ standard deviation of 3 biological replicates (ANOVA, Bonferroni).

Given that aquaporin-5 is a key water channel in salivary gland tissue, like in other exocrine glands [42-44], we can also associate the evaluated product component No. 1 (beta-caryophyllene) with the prevention of xerostomia, stimulation of increased salivary secretion and oral hydration based on the conditions assessed, since it facilitated a 3-fold increase in aquaporin-5 compared to the basal condition.

REFERENCES

1. King, L. S., Yasui, M., & Agre, P. (2000). Aquaporins in health and disease \\ Molecular Medicine Today, 6(2), 60-65.

2. Huber, V. J., Tsujita, M., & Nakada, T. (2012). Aquaporins in drug discovery and pharmacotherapy \\ Molecular Aspects of Medicine, 33(5-6), 691-703.

3. Hara-Chikuma, M., & Verkman, A. S. (2008). Roles of Aquaporin-3 in the Epidermis \\ Journal of Investigative Dermatology, 128(9), 2145-2151.

4. Pelagalli, A.; Squillacioti, C.; Mirabella, N.; Meli, R. Aquaporins in Health and Disease: An Overview Focusing on the Gut of Different Species \\ Int. J. Mol. Sci. (2016), 17, 1213.

5. Snarskaya E.S., Bratkovskaya A.V. Novel filagrinol-containing emollient \\ Vestnik Dermatologii i Venerologii. (2022);98(5):65-89.

6. Tamrazova O.B., Molochkov A.V. Skin xerosi as the main pathogenetic factor in the development of atopic dermatitis \\ Dermatologiya. No. 4, (2014). Pp. 48-54.

7. Coates, M.; Mariottoni, P.; Corcoran, D.L.; Kirshner, H.F.; Jaleel, T.; Brown, D.A.; Brooks, S.R.; Murray, J.; Morasso, M.I.; MacLeod, A.S. The skin transcriptome in hidradenitis suppurativa uncovers an antimicrobial and sweat gland gene signature which has distinct overlap with wounded skin \\ PLoS ONE (2019), 14.

8. Tian, X.; Liu, B.; Chen, L.; Xie, Y.; Liang, J.; Yang, Y.; Shao, L.; Zhang, J.; Wang, J.; Zhang, X.; et al. RNA-Seq Identifies Marked Th17 Cell Activation and Altered CFTR Expression in Different Atopic Dermatitis Subtypes in Chinese Han Populations \\ Front. Immunol. (2021), 12.

9. Nakahigashi, K.; Kabashima, K.; Ikoma, A.; Verkman, A.S.; Miyachi, Y.; Hara-Chikuma, M. Upregulation of aquaporin-3 is involved in keratinocyte proliferation and epidermal hyperplasia \\ J. Invest. Dermatol. (2011), 131, 865-873.

10. Lee, Y.; Je, Y.J.; Lee, S.S.; Li, Z.J.; Choi, D.K.; Kwon, Y.B.; Sohn, K.C.; Im, M.; Seo, Y.J.; Lee, J.H. Changes in transepidermal water loss and skin hydration according to expression of aquaporin-3 in psoriasis \\ Ann. Dermatol. (2012), 24, 168-174.

11. Tamrazova O.B. Dry skin syndrome \\ Dermatologiya. No. 3, (2014). Pp. 8-14.

12. Kazuya Ooi. Onset Mechanism and Pharmaceutical Management of Dry Skin \\ Biol. Pharm. Bull. 44, No. 8. (2021). P. 1037-1043.

13. Gimenez-Arnau, A.M. (2014). Xerosis Means "Dry Skin": Mechanisms, Skin Conditions, and Its Management. In: Thyssen, J., Maibach, H. (eds) Filaggrin. Springer, Berlin, Heidelberg.

14. Gorobets S. M., Romanenko I. G., Dzhereley A. A., Bobkova C. A., Kryuchkov D. Y., Gorobets O. V., Melnichenko D. I. Xerostomia. Modern View On The Problem \\ Tavricheskiy Mediko-Biologicheskiy Vestnik. (2019), vol. 22, No. 2.

P. 83-89.

15. Michael D. Turner, Jonathan A. Ship. Dry Mouth and Its Effects on the Oral Health of Elderly People \\ The Journal of the American Dental Association. V. 138, S. 1, (2007). P. 15-20.

16. Thomson W.M. Dry mouth and older people \\ Aust Dent J. (2015); V. 60, S. 1. P.54-63.

17. Dawes C. How much saliva is enough for avoidance of xerostomia? \\ Caries Res. (2004); 38(3):236-40.

18. Laurence J. Walsh. Dry Mouth: A Clinical Problem for Children and Young Adults \\ International Dentistry SA V. 9, № 5. (2007).

19. Rojek A, Praetorius J, Frøkiaer J, Nielsen S, Fenton R. A current view of the mammalian aquaglyceroporins \\ Annu Rev Physiol (2008); 70: 301-327.

20. Draelos Z. Aquaporins: an introduction to a key factor in the mechanism of skin hydration \\ J Clin Aesthet Dermatol. (2012); 5 (7): 53-56.

21. A. Schrader; W. Siefken; T. Kueper; U. Breitenbach; C. Gatermann; G. Sperling; T. Biernoth; C. Schemer; F. Stäb; H. Wenck; K.-P. Wittern; T. Blatt. Effects of Glyceryl Glucoside on AQP3 Expression, Barrier Function and Hydration of Human Skin \\ Skin Pharmacol Physiol (2012) 25 (4): 192-199.

22. Boury-Jamot M, Daraspe J, Bonté F, Perrier E, Schnebert S, Dumas M, Verbavatz J. Skin aquaporins: function in hydration, wound healing, and skin epidermis homeostasis \\ Handb Exp Pharmacol (2009); 190: 205-217.

23. Zheng X, Bollinger Bollag W. Aquaporin 3 colocates with phospholipase d2 in caveolin-rich membrane microdomains and is downregulated upon keratinocyte differentiation \\ J Invest Dermatol (2003); 121: 1487-1495.

24. Dumas M, Gondran C, Barré P, Sougrat R, Verbavatz JM, Heusele C, Schnébert S, Bonté F. Effect of an Ajuga turkestanica extract on aquaporin 3 expression, water flux, differentiation and barrier parameters of the human epidermis \\ Eur J Dermatol (2002); 12: 25-26.

25. Brandner J. Pores in the epidermis: aquaporins and tight junctions \\ Int J Cosmet Sci (2007); 29: 413-422.

26. Hara-Chikuma M, Takahashi K, Chikuma S, Verkman AS, Miyachi Y. The expression of differentiation markers in aquaporin-3 deficient epidermis \\ Arch Dermatol Res (2009); 301: 245-252.

27. Papadopoulos MC, Saadoun S, Verkman AS. Aquaporins and cell migration \\ Pflugers Arch (2008); 456: 693-700.

28. Itoh K, Kawasaki S, Kawamoto S, Seishima M, Chiba H, Michibata H, Wakimoto K, Imai Y, Minesaki Y, Otsuji M, Okubo K. Identification of differentially expressed genes in psoriasis using expression profiling approaches \\ Exp Dermatol (2005); 14: 667-674.

29. Nejsum LN, Kwon TH, Marples D, Flyvbjerg A, Knepper MA, Frøkiaer J, Nielsen S. Compensatory increase in AQP2, p-AQP2, and AQP3 expression in rats with diabetes mellitus \\ Am J Physiol Renal Physiol (2001); 280: F715-F726.

30. Dumas M, Sadick NS, Noblesse E, Juan M, Lachmann-Weber N, Boury-Jamot M, Sougrat R, Verbavatz JM, Schnebert S, Bonté F. Hydrating skin by stimulating biosynthesis of aquaporins \\ J Drugs Dermatol (2007); 6: 20-24.

31. Elvie Lim Chien Hui, Faith Chuah Ci Kim, Nur Khamalin Nalisa Binti Kamaruddin, A. Thentamil and Mathew Jacob. Aquaporins in Salivary Gland - The Water Fa(u)cet of an Acini? \\ Journal of Academy of Dental Education, (2018), V. 4, I. 1, 12-16.

32. Borok, Z., Li, X., Fernandes, V. F. J., Zhou, B., Ann, D. K., & Crandall, E. D. (2000). Differential Regulation of Rat Aquaporin-5 Promoter/Enhancer Activities in Lung and Salivary Epithelial Cells. Journal of Biological Chemistry, 275(34), 26507-26514.

33. Iizuka, T., Suzuki, T., Nakano, K., & Sueki, H. (2011). Immunolocalization of aquaporin-5 in normal human skin and hypohidrotic skin diseases \\ The Journal of Dermatology, 39(4), 344-349.

34. Soyoung Hwang, Jung Yun Kang, Min Jae Kim, Dong Min Shin & Jeong Hee Hong (2019). Carbonic anhydrase 12 mutation modulates membrane stability and volume regulation of aquaporin 5 \\ Journal of Enzyme Inhibition and Medicinal Chemistry, 34:1, 179-188.

35. Krane, C. M., Melvin, J. E., Nguyen, H.-V., Richardson, L., Towne, J. E., Doetschman, T., & Menon, A. G. (2001). Salivary Acinar Cells from Aquaporin 5-deficient Mice Have Decreased Membrane Water Permeability and Altered Cell Volume Regulation. Journal of Biological Chemistry, 276(26), 23413-23420.

36. Blaydon, D. C., Lind, L. K., Plagnol, V., Linton, K. J., Smith, F. J. D., Wilson, N. J., Kelsell, D. P. (2013). Mutations in AQP5, Encoding a Water-Channel Protein, Cause Autosomal-Dominant Diffuse Nonepidermolytic Palmoplantar Keratoderma \\ The American Journal of Human Genetics, 93(2), 330-335.

37. Tricarico, P.M.; Mentino, D.; De Marco, A.; Del Vecchio, C.; Garra, S.; Cazzato, G.; Foti, C.; Crovella, S.; Calamita, G. Aquaporins Are One of the Critical Factors in the Disruption of the Skin Barrier in Inflammatory Skin Diseases \\ Int. J. Mol. Sci. 2022, 23, 4020.

38. Serge Steinfeld, Elie Cogan, Landon S. King, Peter Agre, Robert Kiss, and Christine Delporte. Abnormal Distribution of Aquaporin-5 Water Channel Protein in Salivary Glands from Sjögren's Syndrome Patients \\ Laboratory Investigation, 2001. V. 81, № 2. P. 143-148.

39. Madeira Ana, Moura Teresa F., Soveral Graca. Detecting Aquaporin Function and Regulation \\ Frontiers in Chemistry (2016). V. 4. P. 2296-2646.

40. Sharma, C., Al Kaabi, J. M., Nurulain, S., Amjad Kamal, M., and Ojha, S. (2016). Polypharmacological Properties and Therapeutic Potential of β-Caryophyllene: A Dietary Phytocannabinoid of Pharmaceutical Promise \\ CPD 22, 3237-3264.

41. Machado, K. da C., Islam, M. T., Ali, E. S., Rouf, R., Uddin, S. J., Dev, S., Melo-Cavalcante, A. A. de C. (2018). A systematic review on the neuroprotective perspectives of beta-caryophyllene \\ Phytotherapy Research. V. 32, I. 12. P. 2376-2388.

42. Matsuzaki T, Susa T, Shimizu K, Sawai N, Suzuki T, Aoki T, Yokoo S, Takata K. Function of the membrane water channel aquaporin-5 in the salivary gland. Acta Histochem Cytochem. 2012 Oct 31;45(5):251-9.

43. Perez P, Rowzee AM, Zheng C, Adriaansen J, Baum BJ. Salivary epithelial cells: an unassuming target site for gene therapeutics. Int J Biochem Cell Biol. 2010 Jun;42(6):773- 7.

44. Hosoi K, Yao C, Hasegawa T, Yoshimura H, Akamatsu T. Dynamics of Salivary Gland AQP5 under Normal and Pathologic Conditions. International Journal of Molecular Sciences. 2020; 21(4):1182.

## Claims

1. A composition comprising 0.01-0.3 wt.% β-caryophyllene.

2. The composition of claim 1 comprising 0.02-0.3 wt.% β-caryophyllene, preferably 0.03-0.25 wt.% β-caryophyllene.

3. The composition of claim 1 or claim 2, wherein said composition is a pharmaceutical composition.

4. The pharmaceutical composition of claim 3, wherein said pharmaceutical composition is formulated for application on a skin and/or mucous membranes continuous with the skin at body openings, including the eyes, eyelids, ears, inside the nose, inside the mouth, lips, the genital areas, the urethral opening and the anus.

5. The composition of claim 1 or claim 2, wherein said composition is for use as a medicament.

6. The composition for use of claim 5, wherein said composition is formulated for application on a skin and/or mucous membranes continuous with the skin at body openings of a subject, including the eyes, eyelids, ears, inside the nose, inside the mouth, lips, the genital areas, the urethral opening and the anus.

7. The composition of claim 1 or claim 2 for use in the treatment of a subject suffering from a disease associated with a deficiency of aquaporins type 3 (AQP 3) and/or aquaporins type 5 (AQP 5), and/or suffering from at least one of the following diseases or symptoms:

   - dry eye and/or dry mouth syndrome;
   - dermatitis, including seborrheic and atopic dermatitis;
   - psoriasis;
   - eczema;
   - vitiligo;
   - xerosis;
   - xerostomia;
   - excessive scaling of the skin, including dandruff of the scalp;
   - proliferative hyperkeratosis;
   - skin itching.

8. The composition for use of claim 7, wherein said composition is formulated for application on a skin and/or mucous membranes continuous with the skin at body openings of a subject, including the eyes, eyelids, ears, inside the nose, inside the mouth, lips, the genital areas, the urethral opening and the anus.

9. The composition of any of claims 1-4 or the composition for use of any of claims 5-8, wherein said formulation is selected from the following:

   - film;
   - aerosol, including aerosol for inhalation;
   - suspension,

- solution,
- tincture,
- cream,
- paste,
- lotion,
- ointment,
- gel,

wherein said formulations are preferably skin and/or mucosa adhesive formulations.

10. The use of the composition of any of claims 1-4 or the composition for use of any of claims 5-9, wherein said composition reduces the moisture loss

- of the skin, preferably by increasing the number of AQP 3 in layers of the skin epidermis; and/or
- of the mucosa, preferably by increasing the number of AQP 5 in the mucosa layers.

11. A body care product comprising the composition of claim 1 or claim 2.

12. The body care product of claim 11, wherein said body care product is selected from the group consisting of:

- cosmetic;
- perfume;
- gel, including shower gel and cleansing gel;
- shampoo, including shampoo for scalp and hair care;
- balm or butter;
- conditioner, including hair conditioner;
- soap, including liquid soap for hand washing;
- cleanser, including facial cleanser;
- cream, including cream for face, body and hand care;
- serum for face care;
- - masks, including cleansing masks;
- solid product for face, hand, and hair care;
- oral care products, including toothpastes, tooth gels, mouthwashes, moisturizing gels, oral serums, resorbable forms, powders.

13. The use of the body care product of claim 11 or claim 12, wherein said body care product is for application to the body's skin and/or mucous membranes continuous with the skin at body openings, including the eyes, eyelids, ears, inside the nose, inside the mouth, lips, the genital areas, the urethral opening and the anus, and wherein the application of said body care reduces the moisture loss

- of the skin, preferably by increasing the number of AQP 3 in layers of the skin epidermis; and/or
- of the mucosa, preferably by increasing the number of AQP 5 in the mucosa layers.

14. Non-medical use of the composition of claim 1 or claim 2, wherein said use of said composition is

i) in cosmetology;
ii) as a component of a body care product, wherein said body care product is preferably selected from the group consisting of:

- cosmetic;
- perfume;
- gel, including shower gel and cleansing gel;
- shampoo, including shampoo for scalp and hair care;
- balm or butter;
- conditioner, including hair conditioner;
- soap, including liquid soap for hand washing;

- cleanser, including facial cleanser;
- cream, including cream for face, body and hand care;
- serum for face care;
- masks, including cleansing masks;
- solid product for face, hand, and hair care;
- oral care products, including toothpastes, tooth gels, mouthwashes, moisturizing gels, oral serums, resorbable forms, powders;

iii) for body and head skin care;
iv) for distribution of transepidermal moisture flow in body and skin cells without disturbing the elastic-viscous-plastic properties of the epidermis cells;
v) regulating transepidermal water flow between cells throughout the epidermis, including promoting a decrease in transepidermal water loss;
vi) reducing transepidermal moisture loss (TEWL) in dehydrated skin.

15. β-caryophyllene or the composition comprising β-caryophyllene of any of claims 1-4 for use in the treatment of a disease associated with a genetic or epigenetic deficiency of AQP 3 and/or AQP 5 in a subject, wherein said β-caryophyllene or said β-caryophyllene comprising composition is for use in epigenetic therapies, preferably for epigenetically increasing the number of AQP 3 and or AQP5 in young keratinocytes of skin and scalp epidermis, mucous epithelium, secretory epithelium, nasal and respiratory mucosa epithelium.

Evaluation of beta-caryophyllene cytotoxicity on keratinocytes

Fig. 1

Determination of AQP3 quantity in skin keratinocytes

Fig. 2

Determination of AQP3 quantity in skin keratinocytes

Fig. 3

Europäisches
Patentamt

European
Patent Office

Office européen
des brevets

## EUROPEAN SEARCH REPORT

**Application Number**

EP 23 22 0681

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/263643 A1 (NEXZOL PHARMA INC [US]) 30 December 2020 (2020-12-30) | 1-15 | INV. |
| Y | * paragraphs [0122], [0125], [0163], [0171] - [0173], [0176], [0218] *  <br> * example 13 * <br> * claims 1-51 * | 1-15 | A61K31/015 <br> A61K8/31 <br> A61P17/00 <br> A61P17/04 <br> A61Q19/00 <br> A61Q19/10 |
| X | US 2022/175703 A1 (BROWN DALE G [US] ET AL) 9 June 2022 (2022-06-09) | 1-15 | |
| Y | * claims 1-18 * <br> * examples 1-9 * | 1-15 | |
| X | CN 108 514 555 A (GUANGZHOU UNIV OF CHINESE MEDICINE GUANGZHOU INSTITUTE OF TRADITIONAL) 11 September 2018 (2018-09-11) | 1-15 | |
| Y | * claims 1-4 * <br> * example 1 * | 1-15 | |
| Y | KESORNNOI BUDSARIN ET AL: "Bael Leaf Oil Increases AQP3 Expression and Exerts Wound-Healing Effect in Human Immortalized Keratinocytes", NARESUAN UNIVERSITY JOURNAL: SCIENCE AND TECHNOLOGY, vol. 31, no. 3, 26 August 2023 (2023-08-26), pages 67-75, XP093172460, ISSN: 2539-553X, DOI: https://doi.org/10.14456/nujst.2023.26 Retrieved from the Internet: URL:https://www.journal.nu.ac.th/NUJST/article/view/Vol-31-No-3-2023-67-75/2223> * abstract * | 1-15 | TECHNICAL FIELDS SEARCHED (IPC) <br><br> A61K <br> A61Q <br> A61P |

-/--

The present search report has been drawn up for all claims

| Place of search | Date of completion of the search | Examiner |
|---|---|---|
| Munich | 11 June 2024 | Hörtner, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| Europäisches Patentamt European Patent Office Office européen des brevets | **EUROPEAN SEARCH REPORT** | Application Number EP 23 22 0681 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 7 December 2021 (2021-12-07), anonymous: "Body Wash", XP093172660, Database accession no. 9219330 * abstract * ----- | 12-14 | |
| | | | TECHNICAL FIELDS SEARCHED (IPC) |

The present search report has been drawn up for all claims

| Place of search | Date of completion of the search | Examiner |
|---|---|---|
| Munich | 11 June 2024 | Hörtner, Michael |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................................
& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 23 22 0681

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2024

| Patent document cited in search report | | Publication date | Patent family member(s) | | Publication date |
|---|---|---|---|---|---|
| WO 2020263643 | A1 | 30-12-2020 | AU | 2020301119 A1 | 20-01-2022 |
| | | | CA | 3144250 A1 | 30-12-2020 |
| | | | EP | 3989959 A1 | 04-05-2022 |
| | | | US | 2022110860 A1 | 14-04-2022 |
| | | | WO | 2020263643 A1 | 30-12-2020 |
| US 2022175703 | A1 | 09-06-2022 | US | 2022175703 A1 | 09-06-2022 |
| | | | US | 2024165057 A1 | 23-05-2024 |
| CN 108514555 | A | 11-09-2018 | CN | 108514555 A | 11-09-2018 |
| | | | WO | 2019237555 A1 | 19-12-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2456977 C1, Demenko V.I., Shcherban V.P.
- RU 2643928 C2

**Non-patent literature cited in the description**

- *CHEMICAL ABSTRACTS*, 87-44-5
- *CHEMICAL ABSTRACTS*, 10579-93-8
- **KING, L. S.** ; **YASUI, M** ; **AGRE, P.** Aquaporins in health and disease \\. *Molecular Medicine Today*, 2000, vol. 6 (2), 60-65
- **HUBER, V. J** ; **TSUJITA, M** ; **NAKADA, T.** Aquaporins in drug discovery and pharmacotherapy. *Molecular Aspects of Medicine*, 2012, vol. 33 (5-6), 691-703
- **HARA-CHIKUMA, M.** ; **VERKMAN, A. S.** Roles of Aquaporin-3 in the Epidermis \\. *Journal of Investigative Dermatology*, 2008, vol. 128 (9), 2145-2151
- **PELAGALLI, A.** ; **SQUILLACIOTI, C** ; **MIRABELLA, N.** ; **MELI, R**. Aquaporins in Health and Disease: An Overview Focusing on the Gut of Different Species. *Int. J. Mol. Sci.*, 2016, vol. 17, 1213
- **SNARSKAYA E.S** ; **BRATKOVSKAYA A.V**. Novel filagrinol-containing emollient. *Vestnik Dermatologii i Venerologii*, 2022, vol. 98 (5), 65-89
- **TAMRAZOVA O.B.** ; **MOLOCHKOV A.V**. Skin xerosi as the main pathogenetic factor in the development of atopic dermatitis. *Dermatologiya*, 2014 (4), 48-54
- **COATES, M** ; **MARIOTTONI, P.** ; **CORCORAN, D.L** ; **KIRSHNER, H.F** ; **JALEEL, T** ; **BROWN, D.A** ; **BROOKS, S.R** ; **MURRAY, J.** ; **MORASSO, M.I.** ; **MACLEOD, A.S**. The skin transcriptome in hidradenitis suppurativa uncovers an antimicrobial and sweat gland gene signature which has distinct overlap with wounded skin. *PLoS ONE*, 2019, vol. 14
- **TIAN, X** ; **LIU, B.** ; **CHEN, L** ; **XIE, Y** ; **LIANG, J.** ; **YANG, Y** ; **SHAO, L** ; **ZHANG, J.** ; **WANG, J.** ; **ZHANG, X et al.** RNA-Seq Identifies Marked Th17 Cell Activation and Altered CFTR Expression in Different Atopic Dermatitis Subtypes in Chinese Han Populations. *Front. Immunol.*, 2021, vol. 12
- **NAKAHIGASHI, K** ; **KABASHIMA, K** ; **IKOMA, A** ; **VERKMAN, A.S** ; **MIYACHI, Y.** Hara-Chikuma, M. Upregulation of aquaporin-3 is involved in keratinocyte proliferation and epidermal hyperplasia. *J. Invest. Dermatol.*, 2011, vol. 131, 865-873
- **LEE, Y** ; **JE, Y.J.** ; **LEE, S.S** ; **LI, Z.J** ; **CHOI, D.K** ; **KWON, Y.B** ; **SOHN, K.C.** ; **IM, M** ; **SEO, Y.J** ; **LEE, J.H**. Changes in transepidermal water loss and skin hydration according to expression of aquaporin-3 in psoriasis. *Ann. Dermatol*, 2012, vol. 24, 168-174
- **TAMRAZOVA O.B.** Dry skin syndrome. *Dermatologiya*, 2014 (3), 8-14
- **KAZUYA OOI.** Onset Mechanism and Pharmaceutical Management of Dry Skin. *Biol. Pharm. Bull.*, 2021, vol. 44 (8), 1037-1043
- **GIMENEZ-ARNAU, A.M**. Xerosis Means "Dry Skin": Mechanisms, Skin Conditions, and Its Management.. Springer, 2014
- **GOROBETS S. M** ; **ROMANENKO I. G** ; **DZHERELEY A. A.** ; **BOBKOVA C. A** ; **KRYUCHKOV D. Y** ; **GOROBETS O. V** ; **MELNICHENKO D. I**. Xerostomia. Modern View On The Problem. *Tavricheskiy Mediko-Biologicheskiy Vestnik*, 2019, vol. 22 (2), 83-89
- **MICHAEL D. TURNER** ; **JONATHAN A. SHIP**. Dry Mouth and Its Effects on the Oral Health of Elderly People. *The Journal of the American Dental Association. V*, 2007, vol. 138 (1), 15-20
- **THOMSON W.M.** Dry mouth and older people. *Aust Dent J.*, 2015, vol. 60 (1), 54-63
- **DAWES C.** How much saliva is enough for avoidance of xerostomia?. *Caries Res*, 2004, vol. 38 (3), 236-40
- **ROJEK A** ; **PRAETORIUS J** ; **FRØKIAER J** ; **NIELSEN S** ; **FENTON R**. A current view of the mammalian aquaglyceroporins. *Annu Rev Physiol*, 2008, vol. 70, 301-327
- **DRAELOS Z**. Aquaporins: an introduction to a key factor in the mechanism of skin hydration. *J Clin Aesthet Dermatol*, 2012, vol. 5 (7), 53-56
- **A. SCHRADER** ; **W. SIEFKEN** ; **T. KUEPER** ; **U. BREITENBACH** ; **C. GATERMANN** ; **G. SPERLING** ; **T. BIERNOTH** ; **C. SCHEMER** ; **F. STÄB** ; **H. WENCK**. Effects of Glyceryl Glucoside on AQP3 Expression, Barrier Function and Hydration of Human Skin. *Skin Pharmacol Physiol*, 2012, vol. 25 (4), 192-199

- **BOURY-JAMOT M** ; **DARASPE J** ; **BONTÉ F** ; **PERRIER E** ; **SCHNEBERT S** ; **DUMAS M** ; **VERBAVATZ J**. Skin aquaporins: function in hydration, wound healing, and skin epidermis homeostasis. *Handb Exp Pharmacol*, 2009, vol. 190, 205-217
- **ZHENG X** ; **BOLLINGER BOLLAG W**. Aquaporin 3 colocates with phospholipase d2 in caveolin-rich membrane microdomains and is downregulated upon keratinocyte differentiation. *J Invest Dermatol*, 2003, vol. 121, 1487-1495
- **DUMAS M** ; **GONDRAN C** ; **BARRÉ P** ; **SOUGRAT R** ; **VERBAVATZ JM** ; **HEUSELE C** ; **SCHNÉBERT S** ; **BONTÉ F**. Effect of an Ajuga turkestanica extract on aquaporin 3 expression, water flux, differentiation and barrier parameters of the human epidermis. *Eur J Dermatol*, 2002, vol. 12, 25-26
- **BRANDNER J.** Pores in the epidermis: aquaporins and tight junctions. *Int J Cosmet Sci*, 2007, vol. 29, 413-422
- **HARA-CHIKUMA M** ; **TAKAHASHI K** ; **CHIKUMA S** ; **VERKMAN AS** ; **MIYACHI Y**. The expression of differentiation markers in aquaporin-3 deficient epidermis. *Arch Dermatol Res*, 2009, vol. 301, 245-252
- **PAPADOPOULOS MC** ; **SAADOUN S** ; **VERKMAN AS**. Aquaporins and cell migration. *Pflugers Arch*, 2008, vol. 456, 693-700
- **ITOH K** ; **KAWASAKI S** ; **KAWAMOTO S** ; **SEISHIMA M** ; **CHIBA H** ; **MICHIBATA H** ; **WAKIMOTO K** ; **IMAI Y** ; **MINESAKI Y** ; **OTSUJI M**. Identification of differentially expressed genes in psoriasis using expression profiling approaches. *Exp Dermatol*, 2005, vol. 14, 667-674
- **NEJSUM LN** ; **KWON TH** ; **MARPLES D** ; **FLYVBJERG A** ; **KNEPPER MA** ; **FRØKIAER J** ; **NIELSEN S**. Compensatory increase in AQP2, p-AQP2, and AQP3 expression in rats with diabetes mellitus. *Am J Physiol Renal Physiol*, 2001, vol. 280, F715-F726
- **DUMAS M** ; **SADICK NS** ; **NOBLESSE E** ; **JUAN M** ; **LACHMANN-WEBER N** ; **BOURY-JAMOT M** ; **SOUGRAT R** ; **VERBAVATZ JM** ; **SCHNEBERT S** ; **BONTÉ F**. Hydrating skin by stimulating biosynthesis of aquaporins. *J Drugs Dermatol*, 2007, vol. 6, 20-24
- **ELVIE LIM CHIEN HUI** ; **FAITH CHUAH CI KIM** ; **NUR KHAMALIN NALISA BINTI KAMARUDDIN**. A. Thentamil and Mathew Jacob. Aquaporins in Salivary Gland - The Water Fa(u)cet of an Acini?. *Journal of Academy of Dental Education*, 2018, vol. 4 (1), 12-16
- **BOROK, Z.** ; **LI, X.** ; **FERNANDES, V. F. J.** ; **ZHOU, B** ; **ANN, D. K** ; **CRANDALL, E. D**. Differential Regulation of Rat Aquaporin-5 Promoter/Enhancer Activities in Lung and Salivary Epithelial Cells. *Journal of Biological Chemistry*, 2000, vol. 275 (34), 26507-26514
- **IIZUKA, T** ; **SUZUKI, T** ; **NAKANO, K** ; **SUEKI, H.** Immunolocalization of aquaporin-5 in normal human skin and hypohidrotic skin diseases. *The Journal of Dermatology*, 2011, vol. 39 (4), 344-349
- **SOYOUNG HWANG** ; **JUNG YUN KANG** ; **MIN JAE KIM** ; **DONG MIN SHIN** ; **JEONG HEE HONG**. Carbonic anhydrase 12 mutation modulates membrane stability and volume regulation of aquaporin 5. *Journal of Enzyme Inhibition and Medicinal Chemistry*, 2019, vol. 34 (1), 179-188
- **KRANE, C. M** ; **MELVIN, J. E** ; **NGUYEN, H.-V** ; **RICHARDSON, L** ; **TOWNE, J. E** ; **DOETSCHMAN, T.** ; **MENON, A. G.** Salivary Acinar Cells from Aquaporin 5-deficient Mice Have Decreased Membrane Water Permeability and Altered Cell Volume Regulation.. *Journal of Biological Chemistry*, 2001, vol. 276 (26), 23413-23420
- **BLAYDON, D. C** ; **LIND, L. K** ; **PLAGNOL, V.** ; **LINTON, K. J** ; **SMITH, F. J. D.** ; **WILSON, N. J.** ; **KELSELL, D. P**. Mutations in AQP5, Encoding a Water-Channel Protein, Cause Autosomal-Dominant Diffuse Nonepidermolytic Palmoplantar Keratoderma. *The American Journal of Human Genetics*, 2013, vol. 93 (2), 330-335
- **TRICARICO, P.M.** ; **MENTINO, D** ; **DE MARCO, A** ; **DEL VECCHIO, C** ; **GARRA, S** ; **CAZZATO, G** ; **FOTI, C** ; **CROVELLA, S** ; **CALAMITA, G**. Aquaporins Are One of the Critical Factors in the Disruption of the Skin Barrier in Inflammatory Skin Diseases. *Int. J. Mol. Sci.*, 2022, vol. 23, 4020
- **MADEIRA ANA** ; **MOURA TERESA F.** ; **SOVERAL GRACA.** Detecting Aquaporin Function and Regulation. *Frontiers in Chemistry*, 2016, vol. 4, 2296-2646
- **SHARMA, C** ; **AL KAABI, J. M** ; **NURULAIN, S** ; **AMJAD KAMAL, M** ; **OJHA, S.** Polypharmacological Properties and Therapeutic Potential of β-Caryophyllene: A Dietary Phytocannabinoid of Pharmaceutical Promise. *CPD*, 2016, vol. 22, 3237-3264
- **MACHADO, K. DA C** ; **ISLAM, M. T** ; **ALI, E. S.** ; **ROUF, R.** ; **UDDIN, S. J.** ; **DEV, S.** ; **MELO-CAVALCANTE, A. A. DE C.** A systematic review on the neuroprotective perspectives of beta-caryophyllene. *Phytotherapy Research*, 2018, vol. 32 (12), 2376-2388
- **MATSUZAKI T** ; **SUSA T** ; **SHIMIZU K** ; **SAWAI N** ; **SUZUKI T** ; **AOKI T** ; **YOKOO S** ; **TAKATA K**. Function of the membrane water channel aquaporin-5 in the salivary gland. *Acta Histochem Cytochem.*, 31 October 2012, vol. 45 (5), 251-9
- **PEREZ P** ; **ROWZEE AM** ; **ZHENG C** ; **ADRIAANSEN J** ; **BAUM BJ**. Salivary epithelial cells: an unassuming target site for gene therapeutics. *Int J Biochem Cell Biol*, June 2010, vol. 42 (6), 773-7

- **HOSOI K** ; **YAO C** ; **HASEGAWA T** ; **YOSHIMURA H** ; **AKAMATSU T**. Dynamics of Salivary Gland AQP5 under Normal and Pathologic Conditions. *International Journal of Molecular Sciences.*, 2020, vol. 21 (4), 1182